# EUROPEAN PATENT APPLICATION

(11) **EP 4 578 944 A1**
(43) Date of publication of application: **02.07.2025**
(21) Application number: 23855982.7
(22) Date of filing: 16.02.2023
(51) Int. Cl.: C12N 9/06, C12N 15/53, C12P 13/04

(54) **AMINOACYL-TRNA SYNTHASE MUTANT AND USE THEREOF**

(30) Priority: 25.08.2022 CN 202211028254
(71) Applicant: Asymchem Laboratories (Tianjin) Co., Ltd., Tianjin 300457 (CN)
(72) Inventor: HONG, Hao, Morrisville North Carolina 27560 (US); JAMES, Gage, Morrisville North Carolina 27560 (US); XIAO, Yi, Tianjin 300457 (CN); ZHANG, Na, Tianjin 300457 (CN); JIAO, Xuecheng, Tianjin 300457 (CN); ZHAO, Junqi, Tianjin 300457 (CN); WANG, Shuyu, Tianjin 300457 (CN); MENG, Xiangyu, Tianjin 300457 (CN)
(74) Representative: Studio Torta S.p.A.
(86) International application number: PCT/CN2023/076493
(87) International publication number: WO 2024/040885

(57) **Abstract**

Provided is an aminoacyl-tRNA synthase mutant and use thereof. The aminoacyl-tRNA synthase mutant is obtained by means of mutation of an amino acid sequence represented by SEQ ID NO: 19, the mutation at least including the following mutation site: I at position 159 is mutated into a hydroxyl-containing amino acid, a basic amino acid or a non-polar amino acid. By means of a method of site-directed mutation, the amino acid sequence is changed, realizing the alteration of the protein structure and function; the mutant has relatively high activity and specificity, so that the mutant can be used for realizing efficient and site-directed introduction of non-natural amino acids in proteins.

## Description

### Technical Field

The disclosure relates to the technical field of enzyme engineering, and in particular, to an aminoacyl tRNA synthetase mutant and use thereof.

### Background

Proteins in organisms are all formed by arranging 20 types of natural amino acids according to gene information, and perform a series of functions. However, the number of functional groups carried by natural amino acids is limited, and thus the natural amino acids cannot meet the requirements of protein structure and function in biological science research and applications. Unnatural amino acids with diverse functional groups have outstanding performance in protein modification. These unnatural amino acids contain ketone group, aldehyde group, azide group, alkynyl, amido, nitro, phosphate, sulfonate, or the like, and can undergo a variety of modification reactions, making them widely used in basic research, drug development, bioengineering and other fields. For example, they are widely used in detecting protein structure changes, antibody-drug conjugates ADC, DNA-peptide conjugation, biosensors, peptide cyclization, fluorescent dye labeling, molecular surface fixation, etc.

The technology of synthetically encoding unnatural amino acids is to introduce unnatural amino acids by adding additional components to the protein translation machinery, including a set of orthogonal tRNAs, aminoacyl tRNA synthetase (aaRS) and amber codon (TAG). The orthogonality of aaRS/tRNA requires that the aaRS cannot recognize endogenous tRNAs or amino acids of a host, and can only be used for aminoacylation of a ligand tRNA thereof, while the tRNAs and target unnatural amino acids cannot be aminoacylated by endogenous aaRS as well. A tyrosyl-tRNA synthase/tRNA pair (MjTyrRS/TyrT) derived from archaea *Methanocaldoccus jannaschii* has been modified and used for the site-directed introduction of various unnatural amino acids, wherein alkynyl in alkynyl-containing unnatural amino acids can perform click chemistry reactions with other molecules containing azide groups, and thus the alkynyl-containing unnatural amino acids can be used for preparing ADC medicaments and protein labelling studies. However, the technology requires multiple rounds of screening based on antibiotics and nuclease barnase, is cumbersome, and does not produce efficient mutants.

### Summary

The disclosure aims to provide an aminoacyl tRNA synthetase mutant and use thereof, so as to improve the introduction efficiency of unnatural amino acids.

In order to achieve the described object, according to one aspect of the disclosure, an aminoacyl tRNA synthase mutant is provided. The aminoacyl tRNA synthase mutant is obtained by mutation of an amino acid sequence as shown in SEQ ID NO: 19, and the mutation includes at least the following mutation site: I at position 159 is mutated to a hydroxyl-containing amino acid, a basic amino acid or a non-polar amino acid.

Furthermore, the mutation at least further comprises one of the following mutation sites: A at position 31 is mutated into a non-polar amino acid; Y at position 32 is mutated to an aromatic or hydroxyl-containing amino acid; E at position 107 is mutated to a negatively-charged amino acid or hydroxyl-containing amino acid; F at position 108 is mutated to a basic amino acid; Q at position 109 is mutated to a basic amino acid; L at position 110 is mutated to methionine; D at position 158 is mutated to an acidic amino acid, hydroxyl-containing amino acid or histidine; H at position 160 is mutated to a basic amino acid, hydroxyl-containing or aromatic amino acid; Y at position 161 is mutated to an aromatic amino acid or a hydroxyl-containing amino acid; or L mutation at position 162 is a hydroxyl-containing amino acid or a non-polar amino acid; and preferably, the mutation comprises at least one of the following combinations of mutation sites:
L69I+E107D+F108R+Q109R+D158H+I159T+H160Q+Y161G+L162T;
A31G+Y32S+E107S+D158T+I159S+H160N+Y161S+L162T; Y32S+L69I+E107S+L110M+I159S;
A31V+Y32T+L65C+F108R+Q109R+L110M+D158H+I159Y+H160S+Y161G+L162M;
A31C+Y32T+E107S+D158S+I159Q+H160F+Y161S+L162T;
Y32S+L65I+E107D+F108K+Q109R+L110M+D158S+I159Q+H160F+Y161G+L162M;
A31L+Y32F+L65I+F108R+L110M+D158T+1159L+H160S+Y161S+L162T; or
A31V+E107S+Q109R+D158E+I159L+Y161W+L162T.

According to another aspect of the disclosure, a DNA molecule is provided. The DNA molecule encodes any of the described aminoacyl tRNA synthase mutants; and preferably, the DNA molecule is obtained by gene mutation of the nucleotide sequence as shown in SEQ ID NO: 7.

According to a further aspect of the disclosure, a recombinant plasmid is provided. The recombinant plasmid comprises any of the described DNA molecules.

Furthermore, the recombinant plasmid is pET-22a(+), pET-22b(+), pET-3a(+), pET-3d(+), pET-11a(+), pET-12a(+), pET-14b(+), pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-18, pUC-18, or pUC-19.

According to a further aspect of the disclosure, a host cell is provided. The host cell comprises any of the described recombinant plasmids.

Further, the host cell comprises a prokaryotic cell; and preferably, the prokaryotic cell is *E*. *coli* BL21-DE3 cells or *E. coli Rosetta-DE3* cells.

According to a further aspect of the disclosure, a method for introducing propynyl-L-tyrosine is provided. The method for introducing propynyl-L-tyrosine includes introducing propynyl-L-tyrosine using any of the described aminoacyl tRNA synthetase mutants or recombinant plasmids; and preferably, includes the following steps: constructing a recombinant plasmid comprising any of the described aminoacyl tRNA synthase mutants and a TyrT gene and a recombinant plasmid comprising a TAG codon at a specific site; and co-transforming the two recombinant plasmids into a host cell, culturing the host cell, and simultaneously adding propynyl-L-tyrosine. More preferably, the host cell is *E. coli;* when the *E. coli* is cultured, the pH of a culture solution is 6.8-7.4, the culture temperature is 16-37°C, and the concentration of propynyl-L-tyrosine is 1-10 mM.

By applying the technical solution of the disclosure, the described transaminoacyl tRNA synthase mutant is mutated by site-directed mutagenesis based on the aminoacyl tRNA synthase as shown in SEQ ID NO: 19, thereby changing its amino acid sequence and achieving changes in protein structure and function. The transaminoacyl tRNA synthase mutant has higher activity and specificity, and thus the use of the mutant can improve the introduction efficiency of unnatural amino acids.

### Brief Description of the Drawings

The accompanying drawings, which form a part of the present application, are used to provide further understanding of the disclosure. The schematic embodiments of the disclosure and the description thereof are used to explain the disclosure, and do not form improper limits to the disclosure. In the drawings:
Fig. 1 shows the construction of a tyrosyl-tRNA synthase mutant library and corresponding amino acid mutation sites; and
Fig. 2 shows the fluorescence expression for unit bacterial concentration.

### Detailed Description of the Embodiments

It is important to note that the embodiments of the present disclosure and the characteristics in the embodiments can be combined under the condition of no conflicts. The present disclosure will be described below with reference to the drawings and embodiments in detail.

The disclosure improved the screening solution based on a green fluorescent protein and a resistance gene in the document (An efficient system for the evolution of aminoacyl-tRNA synthetase specificity Nature Biotechnology volume 20, pages 1044-1048 (2002)), so that the previous screening solution relying on expensive flow cytometry is improved. By replacing the previously dimly luminescent green fluorescent protein with a red fluorescent protein, a screening solution wherein the naked eye can be used for observation was achieved. Under this improved screening platform, we conducted multi-point saturation mutation based on a rational design to address the problem of low efficiency caused by introducing propynyl-L-tyrosine OpY* (the structural formula being shown below). After screening, a mutant with more than 4 times higher introduction efficiency and improved specificity was finally obtained.

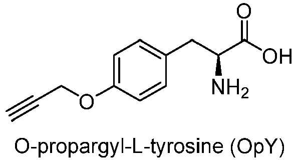

According to a typical embodiment of the disclosure, an aminoacyl tRNA synthase mutant is provided. The aminoacyl tRNA synthase mutant is obtained by mutation of an amino acid sequence as shown in SEQ ID NO: 19, wherein the mutation includes at least the following mutation site: I at position 159 is mutated to a hydroxyl-containing amino acid, a basic amino acid or a non-polar amino acid.

The described transaminoacyl tRNA synthase mutant of the disclosure is mutated by site-directed mutagenesis based on the aminoacyl tRNA synthase as shown in SEQ ID NO: 19, thereby changing its amino acid sequence and achieving changes in protein structure and function. The transaminoacyl tRNA synthase mutant has higher activity and specificity, and thus the use of the mutant can improve the introduction efficiency of unnatural amino acids.

In order to further improve the activity and specificity of the enzyme, the mutation at least further includes one of the following mutation sites: A at position 31 is mutated into a non-polar amino acid; Y at position 32 is mutated to an aromatic or hydroxyl-containing amino acid; E at position 107 is mutated to a negatively-charged amino acid or hydroxyl-containing amino acid; F at position 108 is mutated to a basic amino acid; Q at position 109 is mutated to a basic amino acid; L at position 110 is mutated to methionine; D at position 158 is mutated to an acidic amino acid, hydroxyl-containing amino acid or histidine; H at position 160 is mutated to a basic amino acid, hydroxyl-containing or aromatic amino acid; Y at position 161 is mutated to an aromatic amino acid or a hydroxyl-containing amino acid; or L mutation at position 162 is a hydroxyl-containing amino acid or a non-polar amino acid.

More preferably, the mutation includes at least one of the following combinations of mutation sites: L69I+E107D+F108R+Q109R+D158H+I159T+H160Q+Y161G+L162T; A31G+Y32S+E107S+D158T+I159S+H160N+Y161S+L162T; Y32S+L69I+E107S+L110M+I159S; A31V+Y32T+L65C+F108R+Q109R+L110M+D158H+I159Y+H160S+Y161G+L162M; A31C+Y32T+E107S+D158S+I159Q+H160F+Y161S+L162T; Y32S+L65I+E107D+F108K+Q109R+L110M+D158S+I159Q+H160F+Y161G+L162M; A31L+Y32F+L65I+F108R+L110M+D158T+I159L+H160S+Y161S+L162T; or A31V+E107S+Q109R+D158E+I159L+Y161W+L162T.

According to a typical embodiment of the disclosure, a DNA molecule is provided; preferably, the DNA molecule is obtained by gene mutation based on a nucleotide sequence as shown in SEQ ID NO: 7. The DNA molecule encodes the described aminoacyl tRNA synthase mutant. The described aminoacyl tRNA synthase mutant encoded by the DNA molecule has good specificity and activity.

The described DNA molecule of the disclosure may also be present in the form of "expression cassette". "Expression cassette" refers to a linear or circular nucleic acid molecule, and encompasses DNA and RNA sequences capable of directing expression of a particular nucleotide sequence in an appropriate host cell. Generally, promoters operably ligated to a nucleotide sequence of interest are included, and the promoters are optionally operably ligated to termination signals and/or other regulatory elements. The expression cassette may also includes a sequence required for the correct translation of a nucleotide sequence. The coding region usually encodes a target protein, but it also encodes a functional RNA of interest in the sense or antisense direction, such as antisense RNA or a non-translated RNA. The expression cassette comprising the polynucleotide sequence of interest can be chimeric, meaning that at least one of the components thereof is heterologous to at least one of the other components thereof. The expression cassette may also be naturally occurring, but obtained with efficient recombinant formation for heterologous expression.

According to a typical embodiment of the disclosure, a recombinant plasmid is provided. The recombinant plasmid includes any of the described DNA molecules. The DNA molecule in the described recombinant plasmid is placed at an appropriate position in the recombinant plasmid, so that the described DNA molecule can be correctly and successfully reproduced, transcribed or expressed.

Although the term "comprising" is used in the disclosure to define a DNA molecule, it does not imply that any other sequence not related to its function may be added at either end of the DNA sequence. It is known to a person skilled in the art that, in order to meet the requirements of a recombination operation, it is necessary to add appropriate restriction endonuclease sites at both ends of a DNA sequence, or to add additional a promoter codon, a termination codon, etc. Therefore, if defined by a closed expression, these situations cannot be covered realistically.

The term "plasmid" as used in the disclosure includes any plasmid, cosmid, phage or Agrobacterium binary nucleic acid molecule in double-stranded or single-stranded linear or circular form, and is preferably a recombinant expression plasmid, which can be a prokaryotic expression plasmid or a eukaryotic expression plasmid, but preferably a prokaryotic expression plasmid. In certain embodiments, the recombinant plasmid is selected from pET-22a(+), pET-22b(+), pET-3a(+), pET-3d(+), pET-11a(+), pET-12a(+), pET-14b(+), pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-18, pUC-18, or pUC-19.

To facilitate subsequent screening, a red fluorescent protein reporter gene is operably ligated to the recombinant plasmid; and preferably, the red fluorescent protein reporter gene has a nucleotide sequence as shown in SEQ ID NO: 1. Further, the recombinant plasmid is operably connected to a chloramphenicol gene; and preferably, the recombinant plasmid is operably connected to a promoter and terminator sequence as shown in SEQ ID NO: 6.

According to a typical embodiment of the disclosure, a host cell containing any of the described recombinant plasmids is provided. Suitable host cells for use in the disclosure include, but are not limited to, prokaryotic cells. Preferably, the prokaryotic cell is *E. coli BL21*-DE3 cell or *E. coli Rosetta-DE3* cell.

According to an exemplary embodiment of the disclosure, a method for introducing propynyl-L-tyrosine is provided. The method includes the introduction of propynyl-L-tyrosine using the aminoacyl tRNA synthase mutant or the described recombinant plasmid of the disclosure; and preferably, includes the following steps: constructing a recombinant plasmid containing any of the described aminoacyl tRNA synthase mutants and a TyrT gene and a recombinant plasmid containing a TAG codon at a specific site; and co-transforming the two recombinant plasmids into a host cell, culturing the host cell, and simultaneously adding propynyl-L-tyrosine. More preferably, the host cell is *E. coli*; when the *E. coli* is cultured, the pH of a culture solution is 6.8-7.4, the culture temperature is 16-37°C, and the concentration of propynyl-L-tyrosine is 1-10 mM.

More specifically, in a preferred embodiment of the disclosure, the method for introducing propynyl-L-tyrosine includes: (1) constructing a plasmid containing the MjTyrRS mutant of the disclosure and TyrT, wherein a constitutive promoter is used in the present embodiment, and of course, an inducible promoter such as T7 or Sp6 bacteriophage-derived promoter can also be used; 2) constructing a plasmid expressing a target gene sfGFP (a fluorescent protein used during testing, and the method can also be applied to any other target protein), wherein the T7 induction promoter used can also be replaced with other inducible promoters, including Sp6, xylose induction promoter, arabinose induction promoter, etc.; 3) co-transforming the two plasmids into *E. coli,* wherein BL21 (DE3) is currently used as the host, and the host can also be expanded to any other *E. coli* cells; and 4) cultivating *E. coli,* adding unnatural amino acids at a concentration of 1-10 mM while inducing, and detecting fluorescence after culturing. Preferably, with regard to the plasmid containing a MjTyrRS mutant and TyrT and the plasmid containing a gene of interest, the gene of interest includes one or more TAG mutation sites for introducing a corresponding unnatural amino acid. The two plasmids need to contain different replication initiation sites, so as to ensure that the two plasmids can be stably present at the same time. When the *E. coli* is cultured, the pH is 6.8-7.4, and the culture temperature is 16-37°C. The unnatural amino acid propynyl-L-tyrosine needs to be added. When the unnatural amino acid is dissolved in water, NaOH needs to be added to adjust the pH to about 10.0 for complete dissolution, and then the resultant is filtered and sterilized. When used, the product is added to a medium at a final concentration of 1-10 mM.

The beneficial effects of the disclosure will be further described below in conjunction with the examples.

### Example 1

Construction of a screening protocol based on a red fluorescent protein and chloramphenicol resistance

In the construction of a screening protocol based on a red fluorescent protein and chloramphenicol resistance, specifically, a gene encoding a red fluorescent protein mcherry was used to replace the green fluorescent protein gene GFPuv in the screening plasmid in document (An efficient system for the evolution of aminoacyl-tRNA synthetase specificity Nature Biotechnology volume 20, pages 1044-1048 (2002)). The codon-optimized mcherry sequence was fully synthesized by GENEWIZ, with Ncol restriction enzyme cutting site being introduced at the 5' end of the sequence, and an Xhol restriction enzyme cutting site being introduced at the 3' end of the sequence, and the sequence was constructed in a pET-28a vector. The sequence (SEQ ID NO: 1) is as follows:

ccatgggcATGGTTAGCAAAGGCGAAGAAGATAACATGGCCATCATCAAGGAGTTCATG
CGCTTCAAGGTGCACATGGAGGGCAGCGTGAACGGCCATGAGTTTGAAATCGAGGGCG
AAGGCGAAGGTCGCCCATACGAAGGCACCCAGACCGCCAAACTGAAAGTGACGAAAGG
CGGTCCGCTGCCATTCGCGTGGGATATTCTGAGCCCACAGTTCATGTACGGCAGCAAAGC
CTACGTGAAGCATCCGGCCGATATCCCGGATTATCTGAAGCTGAGCTTCCCAGAGGGCTT
CAAGTGGGAGCGCGTGATGAACTTTGAAGATGGCGGTGTGGTTACCGTTACGCAAGATA
GCAGTCTGCAAGATGGCGAGTTCATCTACAAGGTTAAGCTCCGCGGCACCAACTTCCCG
AGCGATGGTCCGGTGATGCAGAAAAAGACGATGGGCTGGGAAGCGAGCAGCGAACGCA
TGTATCCAGAAGATGGCGCGCTGAAAGGCGAGATCAAACAGCGTCTGAAGCTGAAAGA
CGGCGGCCATTACGATGCGGAGGTGAAGACGACCTACAAAGCCAAGAAACCGGTTCAG
CTGCCGGGCGCCTATAATGTAAACATCAAACTGGATATTACCTCCCACAACGAAGATTAC
ACCATTGTAGAACAATATGAACGCGCGGAAGGCCGCCATAGCACCGGCGGCATGGACGA
ACTGTACAAATAActcgag.

The expression plasmid was transformed into BL21 (DE3) competent cells, and the cells were coated on a solid LB medium plate containing LB + 50 µg/mL kanamycin. After overnight culture, single clones were obtained. The single clones were inoculated into 5 ml of a LB medium, and were cultured at 37°C with shaking at 200 rpm for 2-3 h. When the OD600 was 0.6-0.8, IPTG having a final concentration of 0.5 mM was added, and induction was performed at 30°C for 20 h. It was found that the culture solution became purple, indicating that red fluorescent protein was available. The sequence (Nat Biotechnol. 2002 Oct; 20(10): 1044-1048.), except the green fluorescent protein coding sequence, based on green fluorescence and an antibiotic screening plasmid was amplified by PCR using RFP-HR-Bone-F (SEQ ID NO: 2, GAGTCGTATTAATTTCGCGGGATCGAGTGAGCGCAACGCAATTAATG) and RFP-HR-Bone-R (SEQ ID NO: 3, GCATTAAGCGCGGCGGGTGTGGTGTTTTCACCGTCATCACCG) as primers, and a mcherry coding frame sequence, including a T7 promoter and a T7 terminator, was amplified by PCR using RFP-HR-F: (SEQ ID NO: 4, CATTAATTGCGTTGCGCTCACTCGATCCCGCGAAATTAATACGACTC) and RFP-HR-R: (SEQ ID NO: 5, CGGTGATGACGGTGAAAACACCACACCCGCCGCGCTTAATGC) as primers, and then a plasmid was constructed by homologous recombination, that is, the replacement of the fluorescent protein was completed. The constructed screening plasmid was transformed into DH10B competent cells, and after sequencing, a DH10B strain, named DH10B-REP (screening plasmid), containing the screening plasmid was obtained, and prepared into an electroporation competent cell for use.

### Example 2

### Construction of a pET-Gln constitutive expression plasmid

In order to achieve constitutive expression of aminoacyl tRNA synthase, the promoter and terminator on pET-28a were replaced with Gln promoter and GlnTT terminator. The promoter and terminator were synthesized in a DNA sequence by GENEWIZ. A Bglll (AGATCT) restriction enzyme cutting site was introduced at the 5' end of the promoter, Ndel (CATATG) and EcoRI (GAATTC) sites were introduced at the 3' end, and an Xhol (CTCGAG) site was introduced at the 3' end of the GInTT terminator. The synthesized sequence cut by Bglll and Xhol enzyme was ligated to the pET28a vector. After sequencing, pET-Gln was obtained for use.

The synthesized promoter and terminator sequences are as follows (SEQ ID NO: 6):

AGATCTgagctcccggtcatcaatcatccccataatccttgttagatgatcaattttaaaaaactaacagttcagcctgtcccg
cttataagatccgttatacgtttacgctttgaggaatcccatCATATGGAATTCctgcagtttcaaacgctaaattgcctgatgcgct
acgcttatcaggcctacatgatctctgatatattgagtacgtcttttgtaggccggataatcgttcactcgcatccggcagaaacagcaac
atccaaaacgccgcgttcagcggcgtttatgcttttcttcgcgaattaattccgcttcgcaacatgtgagcaccggtttattgactaccgga
agcagtgtgaccgtgtgctttaaatgcctgaggccagtttgctcaggctctccccgtggaggtaataattgacgatatgatcaCTCGA
G.

### Example 3

### Construction of a tyrosyl-tRNA synthase wild type expression plasmid

Codon-optimized MjTyrRS was synthesized by GENEWIZ, and the sequence was as follows (SEQ ID NO: 7):

ATGGATGAATTTGAAATGATTAAACGCAACACCAGCGAAATTATTAGCGAAGAAGAA
CTGCGCGAAGTGCTGAAGAAGGACGAGAAGTCAGCTTATATTGGCTTTGAACCGAGCGG
CAAGATACACCTGGGCCATTATTTACAGATTAAGAAGATGATCGATTTACAGAACGCGGG

CTTTGATATTATTATTCTGCTGGCGGATCTGCATGCGTATCTGAACCAGAAAGGCGAACTG
GATGAAATTCGCAAGATCGGAGATTATAACAAGAAGGTATTTGAGGCGATGGGCCTGAA
AGCGAAATATGTGTATGGCAGCGAGTTTCAGCTGGATAAAGATTATACCCTGAACGTGTAT
CGCCTGGCGCTGAAGACGACACTGAAACGCGCGCGCCGCAGCATGGAACTGATTGCGC
GCGAAGATGAGAATCCCAAAGTGGCGGAAGTGATTTATCCGATTATGCAGGTGAACGATA
TTCACTATCTGGGCGTGGATGTGGCGGTGGGCGGCATGGAACAGCGCAAGATACACATG
CTGGCGCGCGAACTGCTGCCGAAGAAGGTAGTTTGCATTCATAACCCGGTGCTGACCGG
CCTGGATGGCGAAGGCAAGATGTCCAGCAGCAAAGGCAACTTTATTGCGGTGGATGATA
GCCCGGAAGAAATTCGCGCGAAGATCAAGAAAGCGTACTGCCCGGCGGGCGTGGTGGA
AGGCAACCCGATTATGGAAATTGCGAAATATTTCTTAGAGTATCCGCTGACCATTAAACGC
CCGGAGAAGTTCGGTGGCGATCTGACCGTGAACAGCTATGAAGAACTGGAAAGCCTGTT
TAAGAATAAGGAACTGCATCCGATGGATCTGAAGAATGCTGTGGCGGAAGAACTGATTA
AGATACTCGAACCGATTCGCAAACGCCTGTAA

A pET-Gln-MjTyrRS synthase expression plasmid was obtained by ligating codon-optimized MjTyrRS cut by Bglll and Xhol enzyme to a pET-Gln vector.

The sequence of a MjTyrRS-encoded protein is as follows (SEQ ID NO: 19):

MDEFEMIKRNTSEIISEEELREVLKKDEKSAYIGFEPSGKIHLGHYLQIKKMIDLQNAGFDIIIL
LADLHAYLNQKGELDEIRKIGDYNKKVFEAMGLKAKYVYGSEFQLDKDYTLNVYRLALKT
TLKRARRSMELIAREDENPKVAEVIYPIMQVNDIHYLGVDVAVGGMEQRKIHMLARELLPK
KVVCIHNPVLTGLDGEGKMSSSKGNFIAVDDSPEEIRAKIKKAYCPAGVVEGNPIMEIAKYFL
EYPLTIKRPEKFGGDLTVNSYEELESLFKNKELHPMDLKNAVAEELIKILEPIRKRL.

### Example 4

### Construction of a tyrosyl-tRNA synthase mutant library

Amino acids (31, 32, 65, 67, 69, 107, 108, 109, 110, 158, 159, 160, 161 and 162) on the MjTyrRS that are within 5Å to the para-hydroxy of tyrosine were found to undergo a multi-point saturation mutation by molecular docking. The primers used were as follows:
31-32NNK (SEQ ID NO: 8) : GCTGAAGAAGGACGAGAAGTCANNKNNKATTGGCTTTGAACCGAGCGGCAAGATAC;
65-69-MNN (SEQ ID NO: 9): TCTGGTTCAGATACGCATGMNNATCMNNCAGMNNAATAATAATATCAAAGCCCGCGTTCTG;
65-69NNK (SEQ ID NO: 10): 
GGGCTTTGATATTATTATTNNKCTGNNKGATNNKCATGCGTATCTGAACCAGAAAGGCGAACT
G;
107-110MNN (SEQ ID NO: 11): 
CACGTTCAGGGTATAATCTTTATCMNNMNNMNNMNNGCTGCCATACACATATTTCGCTTTCA
GGCCCATC;
107-110NNK (SEQ ID NO: 12): 
GCGAAATATGTGTATGGCAGCNNKNNKNNKNNKGATAAAGATTATACCCTGAACGTGTATCG
CCTG; and
158-162MNN (SEQ ID NO: 13):

CATGCCGCCCACCGCCACATCCACGCCMNNMNNMNNMNNMNNGTTCACCTGCATAATCG
GATAAATCACTTCCGC.

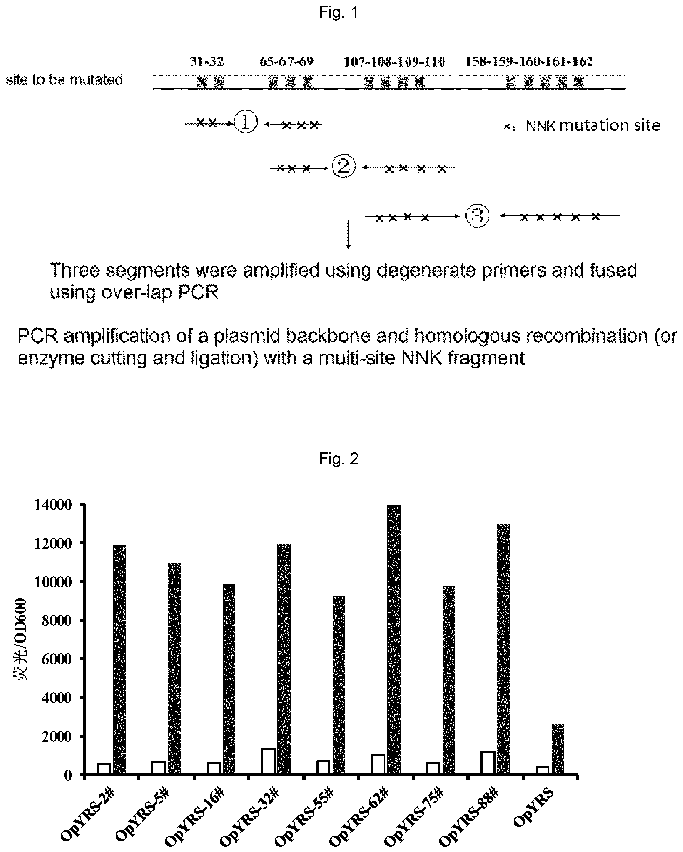

As shown in Figure 1, the primers above were respectively used to amplify the three segments 31-69, 65-110, and 107-162, and then the NNK fragment of MjTyrRS (31-162aa) was obtained by over-lap PCR. The vector part was obtained by PCR amplification using 31-bone-R (SEQ ID NO: 14): TGACTTCTCGTCCTTCTTCAGCACTTCG and 162-bone-F (SEQ ID NO: 15): GGATGTGGCGGTGGGCGGCATG as primers. The NNK fragment and the vector part pET-Gln-MjTyrRS were connected by circular polymerase extension cloning (CPEC) method. The recombinant vector was transformed into DH10B electroporation competent cells by electroporation, and kanamycin was added for overnight culture. The mutant mixed plasmid was extracted using a plasmid extraction kit, and stored at -20°C for later use.

### Example 5

### Tyrosyl tRNA synthase mutant screening

The tyrosyl-tRNA synthase mutant obtained in Example 4 was electrotransformed into DH10B-REP, and after recovery at 37°C for 1 hour, DH10B-REP was coated on an LB solid screening plate (hereinafter referred to as B plate) containing 10 µg/mL tetracycline, 50 µg/mL kanamycin, 50 µg/mL chloramphenicol, 0.1% arabinose, and 1 mM OpY for culturing for 48 h to 72 h until red clones grew out. The red clones were cultured on the B plate and a C plate (1 mM OpY on the B plate was removed) at the same time, and the clones that did not grow on the C plate but grew on the B plate and had red fluorescence were selected and the B plate was streaked. After the red clones grew, 2 to 3 rounds of screening were further performed on the B and C plates, and finally a tyrosyl-tRNA synthase mutant that could efficiently introduce OpY was obtained. The mutant was sequenced to obtain the coding sequence and mutation site. The information is shown in Table 1.

**Table 1**

| MyTyrR | 31A | 32Y | 65L | 67A | 69L | 107E | 108F | 109Q | 110L | 158D | 159I | 160H | 161Y | 162L |
|---|---|---|---|---|---|---|---|---|---|---|---|---|---|---|
| OpYRS-2# | A | Y | L | A | I | D | R | R | L | H | T | Q | G | T |
| OpYRS-5# | G | S | L | A | L | S | F | Q | L | T | S | N | S | T |
| OpYRS-16# | A | S | L | A | I | S | F | Q | M | D | S | H | Y | L |
| OpYRS-32# | V | T | C | A | L | E | R | R | M | H | Y | S | G | M |
| OpYRS-55# | C | T | L | A | L | S | F | Q | L | S | Q | F | S | T |
| OpYRS-62# | A | S | I | A | L | D | K | R | M | S | Q | F | G | M |
| OpYRS-75# | L | F | I | A | L | E | R | Q | M | T | L | S | S | T |
| OpYRS-88# | V | Y | L | A | L | S | F | R | L | E | L | H | W | T |

### Example 6

### Construction of a fluorescent protein recombinant plasmid

A codon-optimized green fluorescent protein sfGFP gene (for testing) was synthesized by GENEWIZ and constructed at the Ncol and Xhol sites of pACYCduet1. The sfGFP gene sequence is as follows (SEQ ID NO: 16):

ATGAGCAAAGGTGAAGAACTGTTTACCGGCGTTGTGCCGATTCTGGTGGAACTGGATGG
CGATGTGAACGGTCACAAATTCAGCGTGCGTGGTGAAGGTGAAGGCGATGCCACGATTG
GCAAACTGACGCTGAAATTTATCTGCACCACCGGCAAACTGCCGGTGCCGTGGCCGACG
CTGGTGACCACCCTGACCTATGGCGTTCAGTGTTTTAGTCGCTATCCGGATCACATGAAA
CGTCACGATTTCTTTAAATCTGCAATGCCGGAAGGCTATGTGCAGGAACGTACGATTAGC

TTTAAAGATGATGGCAAATATAAAACGCGCGCCGTTGTGAAATTTGAAGGCGATACCCTG
GTGAACCGCATTGAACTGAAAGGCACGGATTTTAAAGAAGATGGCAATATCCTGGGCCAT
AAACTGGAATACAACTTTAATAGCCATAATGTTTATATTACGGCGGATAAACAGAAAAATG
GCATCAAAGCGAATTTTACCGTTCGCCATAACGTTGAAGATGGCAGTGTGCAGCTGGCAG
ATCATTATCAGCAGAATACCCCGATTGGTGATGGTCCGGTGCTGCTGCCGGATAATCATTA
TCTGAGCACGCAGACCGTTCTGTCTAAAGATCCGAACGAAAAAGGCACGCGGGACCAC
ATGGTTCTGCACGAATATGTGAATGCGGCAGGTATTACGTGGAGCCATCCGCAGTTCGAA
AAA.

In order to introduce an unnatural amino acid at a specific site in sfGFP, a triple codon encoding I39 thereof was mutated from ATT to TAG, and DNA sequencing was performed to obtain a pACYCduet-sfGFP(I39) plasmid.

### Example 7

### Construction of a plasmid containing the MjTyrRS mutant and TyrT

A coding sequence of TyrT, including the promoter and terminator, was amplified from the screening plasmid REP using tRNA-Xhol-F (SEQ ID NO: 17, gggCTCGAGCCCATCAAAAAAATATTCTCAAC) and tRNA-HR-Xhol-R (SEQ ID NO: 18, gggCTCGAGtaaaaaaaatccttagctttcg). The product was digested with Xhol and ligated with pET-Gln-MjTyrRS (Confirmed by sequencing). The PET-Gln-MyTyrRS(mut)-tRNA plasmid was confirmed by DNA sequencing.

### Example 8

### Evaluation of propynyl-L-tyrosine Introduction

Each of pET-Gln-MyTyrRS(2#)-tRNA, pET-Gln-MyTyrRS(5#)-tRNA, pET-Gln-MyTyrRS(16#)-tRNA, pET-Gln-MyTyrRS(32#)-tRNA, pET-Gln-MyTyrRS(55#)-tRNA, pET-Gln-MyTyrRS(62#)-tRNA, pET-Gln-MyTyrRS(75#)-tRNA, and pET-Gln-MyTyrRS(88#)-tRNA were co-transformed with pACYCduet-sfGFP(I39TAG) into BL21 (DE3) competent cells, and the competent cells were coated on a plate containing kanamycin and chloramphenicol, and cultured at 37°C until single clones grew. Single clones were selected and inoculated in 30 ml LB (kan + Cm + 1 mM OpY), and LB without OpY (kan + Cm) was used as a negative control. After induction with 1 mM IPTG at 30°C overnight, the sample and positive control bacteria were emerald green, indicating good expression. No fluorescence was observed by naked eye in the negative control. 485 nm was used as excitation light and 525 nm was used as emission light to measure the fluorescence intensity of the green fluorescent protein. OD600 was also measured, and the fluorescence value per unit bacterial concentration was calculated. The mutant synthase OpYRS (32A, 107P, 158A, and 162A) reported by document was compared, and the results are shown in Figure 2.

Compared with the OpYRS in the document (In vivo incorporation of an alkyne into proteins in Escherichia coli Bioorganic & Medicinal Chemistry Letters 15 (2005) 1521-1524), the activity of the mutants OpYRS-2#, OpYRS-5#, OpYRS-16#, OpYRS-32#, OpYRS-55#, OpYRS-62#, OpYRS-75#, and OpYRS-88# obtained in the present disclosure was increased by 4.97, 4.57, 4.10, 5.0, 3.85, 5.83, 4.07, and 5.42 times, respectively, and the specificity was also significantly improved (fluorescence value/OD600 when OpY was added: fluorescence value/OD600 without OpY addition), with OpYRS-2# = 20.46, OpYRS-5# = 16.9, OpYRS-16# = 15.8, OpYRS-32# = 9.1, OpYRS-55# = 13.2, OpYRS-62# = 13.5, OpYRS-75# = 16.2, and OpYRS-88# = 10.8, while the control was only 5.72.

From the description above, it can be determined that the described examples of the disclosure achieve the following technical effects:
The disclosure optimizes the screening solution based on red fluorescent light and antibiotic resistance, and realizes a visually recognizable clone screening. Based on the results of the structure and molecular docking, a library of tyrosyl-tRNA synthase mutants covering 14 sites was constructed, and 8 synthase mutants that can efficiently introduce OpY were obtained based on the improved screening solution. Compared with the document, the new synthase mutants obtained have significantly improved activity and specificity.

The description above is only the preferred embodiments of the disclosure, and is not intended to limit the disclosure. For a person skilled in the art, the disclosure may have various modifications and variations. Any modifications, equivalent replacements, improvements and the like made within the spirit and principle of the disclosure shall fall within the scope of protection of the disclosure.

## Claims

1. An aminoacyl tRNA synthetase mutant obtained by mutation of an amino acid sequence as shown in SEQ ID NO: 19, wherein the mutation comprises at least the following mutation site: I at position 159 is mutated into a hydroxyl-containing amino acid, a basic amino acid or a non-polar amino acid.

2. The aminoacyl tRNA synthetase mutant according to claim 1, wherein the mutation at least further comprises one of the following mutation sites: A at position 31 is mutated into a non-polar amino acid; Y at position 32 is mutated to an aromatic or hydroxyl-containing amino acid; E at position 107 is mutated to a negatively-charged amino acid or hydroxyl-containing amino acid; F at position 108 is mutated to a basic amino acid; Q at position 109 is mutated to a basic amino acid; L at position 110 is mutated to methionine; D at position 158 is mutated to an acidic amino acid, hydroxyl-containing amino acid or histidine; H at position 160 is mutated to a basic amino acid, hydroxyl-containing or aromatic amino acid; Y at position 161 is mutated to an aromatic amino acid or a hydroxyl-containing amino acid; or L mutation at position 162 is a hydroxyl-containing amino acid or a non-polar amino acid;
preferably, the mutation comprises at least one of the following combinations of mutation sites:
L69I+E107D+F108R+Q109R+D158H+I159T+H160Q+Y161G+L162T;
A31G+Y32S+E107S+D158T+I159S+H160N+Y161S+L162T; Y32S+L69I+E107S+L110M+I159S;
A31V+Y32T+L65C+F108R+Q109R+L110M+D158H+I159Y+H160S+Y161G+L162M;
A31C+Y32T+E107S+D158S+I159Q+H160F+Y161S+L162T;
Y32S+L65I+E107D+F108K+Q109R+L110M+D158S+I159Q+H160F+Y161G+L162M;
A31L+Y32F+L65I+F108R+L110M+D158T+I159L+H160S+Y161S+L162T; or
A31V+E107S+Q109R+D158E+I159L+Y161W+L162T.

3. A DNA molecule encoding the aminoacyl tRNA synthetase mutant according to any one of claims 1 to 2;
preferably, the DNA molecule is obtained by gene mutation of the nucleotide sequence as shown in SEQ ID NO: 7.

4. A recombinant plasmid comprising the DNA molecule according to claim 3.

5. The recombinant plasmid according to claim 4, wherein the recombinant plasmid is pET-22a(+), pET-22b(+), pET-3a(+), pET-3d(+), pET-11a(+), pET-12a(+), pET-14b(+), pET-15b(+), pET-16b(+), pET-17b(+), pET-19b(+), pET-20b(+), pET-21a(+), pET-23a(+), pET-23b(+), pET-24a(+), pET-25b(+), pET-26b(+), pET-27b(+), pET-28a(+), pET-29a(+), pET-30a(+), pET-31b(+), pET-32a(+), pET-35b(+), pET-38b(+), pET-39b(+), pET-40b(+), pET-41a(+), pET-41b(+), pET-42a(+), pET-43a(+), pET-43b(+), pET-44a(+), pET-49b(+), pQE2, pQE9, pQE30, pQE31, pQE32, pQE40, pQE70, pQE80, pRSET-A, pRSET-B, pRSET-C, pGEX-5X-1, pGEX-6p-1, pGEX-6p-2, pBV220, pBV221, pBV222, pTrc99A, pTwin1, pEZZ18, pKK232-18, pUC-18, or pUC-19.

6. A host cell comprising the recombinant plasmid according to claim 4 or 5.

7. The host cell according to claim 6, wherein the host cell comprises prokaryotic cells; preferably, the prokaryotic cells are *Escherichia coli* BL21-DE3 cells or *Escherichia coli Rosetta-*DE3 cells.

8. A method for introducing propynyl-L-tyrosine, comprising introducing propynyl-L-tyrosine by using the aminoacyl tRNA synthetase mutant according to claim 1 or 2 or the recombinant plasmid according to claim 4 or 5.

9. The method for introducing propynyl-L-tyrosine according to claim 8, wherein the method comprises the following steps:
constructing the recombinant plasmid according to claim 4 or 5 and a recombinant plasmid comprising a TyrT gene; and
co-transforming the two recombinant plasmids into a host cell, culturing the host cell, and simultaneously adding propynyl-L-tyrosine.

10. The method for introducing propynyl-L-tyrosine according to claim 9, wherein the host cell is *Escherichia coli*; when the *Escherichia coli* is cultured, the pH of a culture solution is 6.8-7.4, the culture temperature is 16-37°C, and the concentration of propynyl-L-tyrosine is 1-10 mM.
